# EUROPEAN PATENT SPECIFICATION

(11) **EP 4 190 293 B1**
(45) Date of publication and mention of the grant of the patent: **03.07.2024**
(21) Application number: 21211738.6
(22) Date of filing: 01.12.2021
(51) Int. Cl.: A61F 13/49, A61F 13/496, A61F 13/84, A61F 13/53

(54) **A WASHABLE ABSORBENT GARMENT, METHOD AND APPARATUS FOR PRODUCING THE SAME**
WASCHBARES ABSORBIERENDES KLEIDUNGSSTÜCK, VERFAHREN UND VORRICHTUNG ZUR HERSTELLUNG DAVON
VÊTEMENT ABSORBANT LAVABLE, PROCÉDÉ ET APPAREIL DE PRODUCTION ASSOCIÉS

(43) Date of publication of application: 07.06.2023
(73) Proprietor: Fameccanica.Data S.p.A., 66020 San Giovanni Teatino (CH) (IT)
(72) Inventor: D'APONTE, Francesco, I-66020 San Giovanni Teatino (Chieti) (IT); GUALTIERI, Diego, I-66020 San Giovanni Teatino (Chieti) (IT)
(74) Representative: Marchitelli, Mauro

(56) References cited:
- EP-A1- 2 433 600
- EP-A1- 3 895 675
- EP-A2- 2 412 353
- CN-A- 109 512 581
- CN-A- 109 620 553
- US-A1- 2018 014 983
- US-A1- 2021 290 447

## Description

### Field of the invention

The present invention relates to absorbent garments for the absorption and retention of liquids, and more particularly to washable and reusable absorbent garments.

The invention also relates to a method and apparatus for producing washable absorbent garments.

### Prior art

Various washable garments having a pant-like configuration and useful as incontinence garments, menstrual garments, training garments, diapers, and the like, are commercially available, as well as being disclosed in various patents.

For instance, absorbent garments are commercially available which are machine washable, and thus reusable, having an absorbent pad sewn into the crotch area.

US 3489149 discloses a menstrual panty having a pocket sewn into the crotch area, the pocket being used for receiving an absorbent disposable menstrual pad.

Similarly, US 4352356 discloses a urinary incontinence panty having a pouch connected in the crotch area and adapted to receive an absorbent disposable urinary incontinence pad. Another sanitary panty garment with a pocket in the crotch area is disclosed in US 3613686.

Also, washable absorbent garments are known wherein the absorbent pad is permanently connected to the pant structure. For instance, GB2176692A discloses an absorbent sanitary garment made in the form of knitted polyester material which incorporates a non-removable pad made of brushed polyester which has the appearance of soft felt. The absorbent pad cannot be removed and is made of a material which can be frequently washed.

Washable absorbent garments reduce the environmental impact generated by disposing as waste disposable absorbent sanitary articles after each use and are therefore a sustainable and cost-effective alternative to disposable absorbent sanitary articles.

In order to provide advantages from the point of view of sustainability and cost reduction, washable absorbent garments should be reusable after washing for a relatively high number of times.

One of the problems that limits the diffusion of washable absorbent garments as compared to disposable absorbent sanitary articles is the limited reusability. As a matter of fact, limited reusability may nullify the sustainability benefits and may lead to an increase of costs as compared to the use of disposable absorbent sanitary articles.

One of the factors limiting reusability of washable absorbent garments is the presence of bacteria and unpleasant odors that remain after washing.

### Object and summary of the invention

The object of the present invention is to provide a washable absorbent garment which overcomes the problems of the prior art.

More specifically, the object of the present invention is to provide washable absorbent garments having an improved reusability as compared to the prior art.

According to the present invention, this object is achieved by a washable absorbent garment having the features of claims 1.

According to another aspect, the invention relates to a method and apparatus for producing washable absorbent garments having the features of claims 12 and 14.

The claims form an integral part of the disclosure provided here in relation to the invention.

### Brief description of the drawings

The present invention will now be described in detail with reference to the attached drawings, given purely by way of non-limiting example, wherein:
- Figure 1 is a perspective view of a washable absorbent garment in an open configuration,
- Figure 2 is a plan view of the absorbent garment of figure 1 in the open configuration,
- Figure 3 is a schematic cross-section taken along the line III-III of figure 2,
- Figures 4 and 5 are schematic side views of an apparatus for producing the washable absorbent garment of figures 1-3,
- Figures 6-11 are schematic plan views showing intermediate steps of a method for producing washable absorbent garments, and
- Figure 12 is a schematic side view showing an alternative to the part of the apparatus shown in figure 5.

It will be appreciated that the various figures may not be represented on the same scale. It will also be appreciated that in some figures certain elements or components may not be shown for a better understanding.

### Detailed description

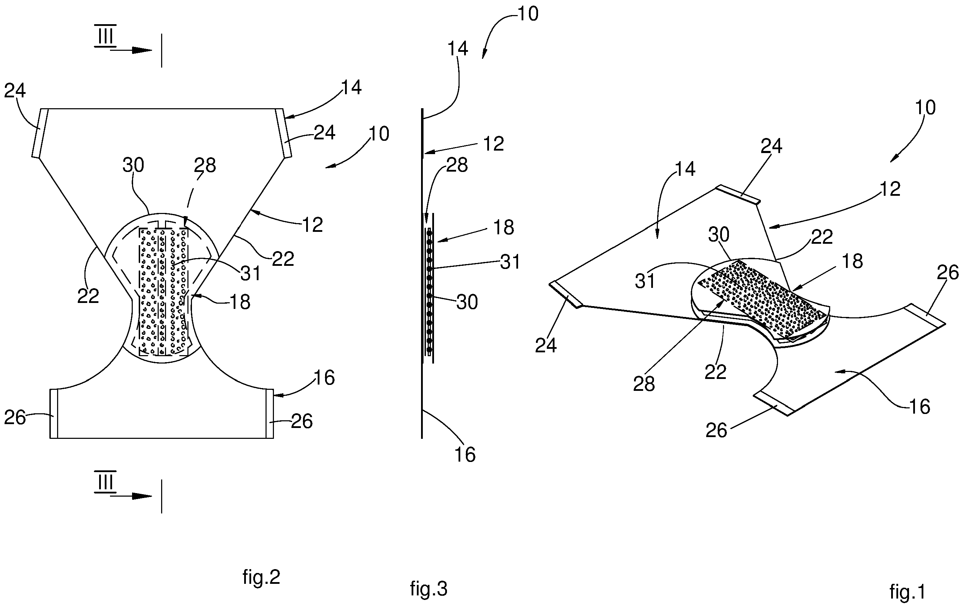

With reference to figures 1-3 the numeral reference 10 indicates a washable absorbent garment.

The washable absorbent garment 10 comprises a back layer 12 of washable fabric forming a pant structure. The back layer 12 has rear and front waist sections 14, 16 and a crotch section 18 intermediate between the rear and front waist sections 14, 16. The rear and front waist sections 14, 16 are wider than the crotch section 18, such that in the extended position of figures 1 and 2 the back layer 12 has substantially an hourglass shape.

The back layer 12 is a fabric formed by woven threads which is washable as ordinary textile garments, e.g. swimsuits, underwear, etc. The back layer 12 may be made of any natural or synthetic material used for producing textile articles, e.g. cotton, Lycra^{®}, nylon, polyester, polypropylene, polyamide, elastane, Econyl^{®} etc. and any mixture thereof. In possible embodiments, the back layer 12 may be an elastic fabric made of a mixture of polyamide and elastane, cotton and elastane e.g. 80% polyamide/cotton and 20% elastane. The back layer 20 may have a weight comprised between 100-200 g/m².

The crotch section 18 of the back layer 12 has two curved side edges 22 shaped to conform to the legs of the user in the configuration in which the washable absorbent garment 10 is worn. The rear and front waist sections 14, 16 of the back layer 12 have respective side edges 24, 26 which are joined to each other such that the washable absorbent garment 10 is closed in a pant-like shape and has two leg openings on opposite sides of the crotch section 18.

The side edges 24, 26 are joined to each other by welding or by glue. Welding may be ultrasonic welding, laser welding, or mechanical welding.

The washable absorbent garment 10 comprises a washable absorbent core 28 permanently fixed in the crotch section 18 of the back layer 12. The washable absorbent core 28 extends only on the crotch section 18 of the back layer 12 and does not extend on the rear and front waist sections 16, 18 of the back layer 12.

The washable absorbent core 28 includes a washable absorbent pad comprising washable superabsorbent fibers. A material suitable for producing the washable absorbent pad 30 may be the one marketed by Technical Absorbents Ltd (UK) under the trade name SAF^{®}. The washable absorbent pad may be formed by needlefelt, thermal bonded, laminated non-woven fibers, formed by polyester, polypropylene and SAF^{®}. The washable absorbent pad may have a weight comprised between 100 and 700 g/m² and a thickness comprised between 1 and 7 mm.

The washable absorbent core 28 may include an acquisition and diffusion layer (ADL) and a barrier film. The washable absorbent pad may be sandwiched between the acquisition and diffusion layer and the barrier film.

The washable absorbent core 28 may have a total weight comprised between 150 and 750 g/m² and a total thickness comprised between 1,5 and 8,5 mm.

The washable absorbent garment 10 comprises a top layer 30 of washable fabric which covers the washable absorbent core 28. The top layer 30 may be made of the same material as the fabric forming the back layer 12. The top layer 30 completely covers the washable absorbent core 28 but does not extend on the rear and front waist sections 16, 18 of the back layer 12.

The top layer 30 and the washable absorbent core 28 are permanently joined to the back layer 12 by welding or by glue.

The washable absorbent core 28 comprises a functionalizing material 31 selected from a group consisting of: graphene, graphene oxide, reduced graphene oxide, and combinations thereof.

The functionalizing material 31 is bacteriostatic but may also be bactericidal. The functionalizing material 31 may be embedded in polymers.

In a possible embodiment, the functionalizing material 31 may be uniformly distributed through the thickness of the washable absorbent core 28. The weight of the functionalizing material 31 distributed in the washable absorbent core 28 may be comprised between 10-250 g/m².

In a possible embodiment, the functionalizing material may be localized on the surface of the washable absorbent core 28 adjacent to the top layer 30. When the functionalizing material is localized on the surface of the washable absorbent core 28 the weight may be comprised in the range 2-100 g/m².

Graphene is a material consisting of a monoatomic layer of carbon atoms, which can be produced by mechanical exfoliation of graphite, liquid phase exfoliation, graphene oxide reduction or by chemical treatment of graphite. Graphene and its derivates are used in various due to their characteristics of mechanical strength, electrical conductivity, and ability to capture particles, bacteria, etc.

Graphene provides antibacterial, antimicrobial, fungicidal and antiviral characteristics to the materials on which it is applied. The washable absorbent garment 10 containing graphene, graphene oxide, reduced graphene oxide, and combinations thereof has therefore better antibacterial, antimicrobial, fungicidal and antiviral and characteristics than prior art washable absorbent garments.

The presence of graphene in the washable absorbent core 28 prevents odors, bacteria, and other biological materials from remaining after washing, which significantly increases the reusability of the washable absorbent garment 10 as compared to prior art washable absorbent garments.

Particularly effective in increasing the reusability of the washable absorbent garment 10 is the odor-control action of graphene in the areas where there is a grater accumulation of body fluids.

The presence of graphene, graphene oxide, and/or reduced graphene oxide, makes the washable absorbent core 28 antistatic, which prevents accumulation of electric charges which can lead to small electric shocks, especially when the back layer 12 includes synthetic fibers.

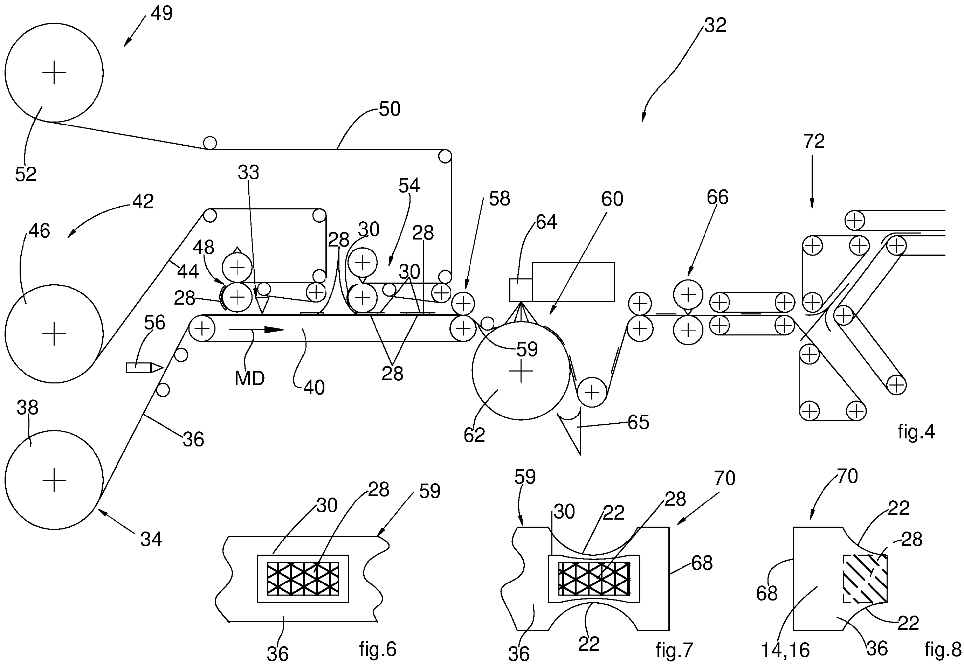

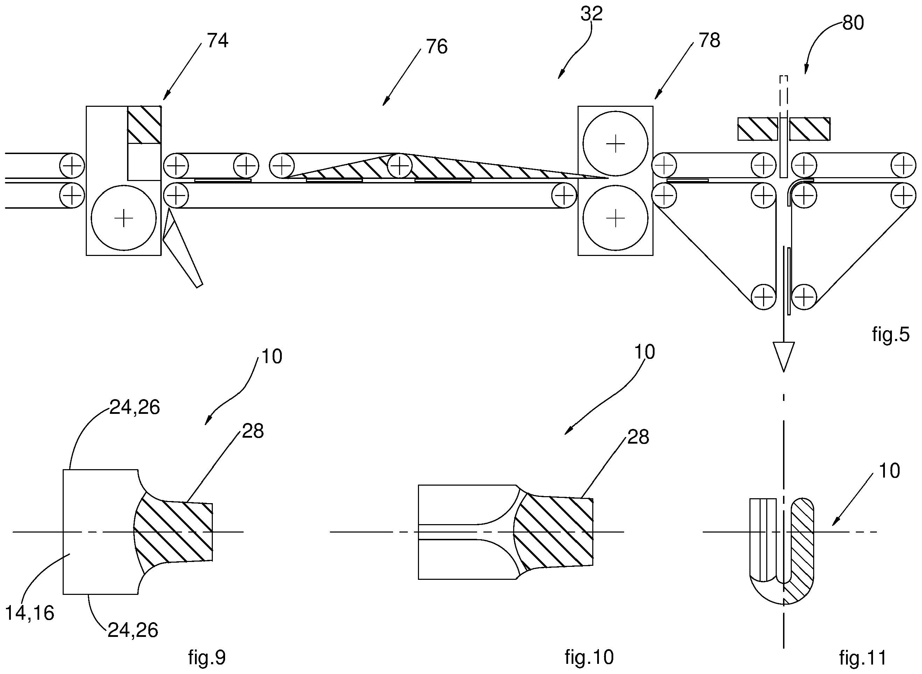

An embodiment of an apparatus for producing the washable absorbent garment 10 of the type illustrated in Figures 1-3 is shown in figures 4 and 5 and is indicated by the reference numeral 32.

With reference to figure 4, the apparatus 32 comprises a first unwinding unit 34 configured for unwinding a first continuous fabric layer 36 from a first reel 38. The first continuous fabric layer 36 is advanced in a machine direction MD on a conveyor 40.

The apparatus 32 comprises a second unwinding unit 42 configured for unwinding a continuous absorbent tape 44 from a second reel 46. The continuous absorbent tape 44 may comprise a web of superabsorbent fibers sandwiched between an acquisition and diffusion layer and a barrier layer. Alternatively, an acquisition and diffusion layer and a barrier layer may be applied in-line on opposite sides of a web of superabsorbent fibers unwound from the second reel 46. The web of superabsorbent fibers may be sandwiched between two soft non-woven layers.

The apparatus 32 comprises an application device 33 configured for applying a functionalizing material 31 selected among graphene, graphene oxide, reduced graphene oxide, and combinations thereof, to the continuous washable absorbent tape 44. The functionalizing material 31 may be applied to the continuous washable absorbent tape 44 in the form of powder or in the form of a liquid suspension.

The apparatus 32 comprises a first cut-and-slip unit 48 configured for transversally cutting the continuous absorbent tape 44 to form individual absorbent cores 28. The first cut-and-slip unit 48 is configured for longitudinally spacing from each other the individual absorbent cores 28 and for applying the individual absorbent cores 28 on the first continuous fabric layer 36 in longitudinally spaced positions on the conveyor 40.

The apparatus 32 comprises a third unwinding unit 49 configured for unwinding a second continuous fabric layer 50 from a third reel 52. The apparatus 32 comprises a second cut-and-slip unit 54 configured for transversally cutting the second continuous fabric layer 50 to form individual top layers 30. The second cut-and-slip unit 54 is configured for longitudinally spacing from each other the individual top layers 30 and for applying the individual top layers 30 on respective absorbent cores 28 previously applied on the first continuous fabric layer 36 on the conveyor 40.

The apparatus 32 may comprise a glue dispenser 56 configured for applying a discrete pattern of glue on the first continuous fabric layer 36 for permanently attaching by glue the individual absorbent cores 28 and the individual top layers 30 on the first continuous fabric layer 36.

The apparatus 32 comprises a sealing press 58 located at the end of the conveyor 40, configured for compressing the individual absorbent cores 28 and the individual top layers 30 to the first continuous fabric layer 36. After the compression, the individual absorbent cores 28 and the individual top layers 30 are permanently fixed by glue to the first continuous fabric layer 36 in longitudinally spaced positions to form a continuous garment tape 59.

Alternatively, the apparatus 32 may comprise a welding unit configured for permanently attaching the individual absorbent cores 28 and the individual top layers 30 on the first continuous fabric layer 36. The welding unit may be a ultrasonic or mechanical welding unit.

Figure 6 shows a portion of the continuous garment tape 59 formed by individual absorbent cores 28 and individual top layers 30 fixed to the first continuous fabric layer 36.

The apparatus 32 comprises a cutting unit 60 configured for cutting longitudinal side edges of the continuous garment tape 59 as it advances in the machine direction MD along curved profiles shaped to conform to the legs of the user. The cutting unit 60 comprises an anvil roller 62 and a cutting tool 64 cooperating with the outer surface of the anvil roller 62. The cutting tool 64 may be a laser beam or an ultrasonic horn configured for carrying out a laser or ultrasonic cut of the first continuous fabric layer 36. The cutting tool 64 may also cut simultaneously side edges of the top layers 30.

The cutting tool 64 may be movable transversally to the machine direction to form, in conjunction with the longitudinal movement of the continuous garment tape 59, curved cuts which form curved side edges 22 (figure 7) of the continuous garment tape 59. The portions trimmed from the continuous garment tape 59 are collected in a waste collecting channel 65. The same results could be obtain employing traditional mechanical rotary cutting devices, but with very high limitation with respect to process flexibility and cost.

The apparatus 32 may comprise a press unit followed by a transverse cutting unit 66 configured to cut the first continuous fabric layer 36 along transverse cutting lines 68 (figure 7) to form individual blank absorbent garments 70. Figure 7 shows the continuous garment tape 59 after the longitudinal and transverse cutting steps.

The apparatus 32 may comprise a transverse folding unit 72 configured for folding the individual blank absorbent garments 70 along respective transverse folding lines, so as to overlap to each other the rear and front waist sections 14, 16 of each blank absorbent garment 70. Figure 8 shows a blank absorbent garment 70 after the transverse folding step.

With reference to figure 5, the apparatus 32 comprises a sealing unit 74 which receives the folded blank absorbent garments 70 from the folding unit 72. With reference to figure 9, the sealing unit 74 is configured for sealing side edges 24, 26 of the rear and front waist sections 14, 16 of the blank absorbent garments 70, to form finished absorbent garments 10. The sealing unit 74 may be an ultrasonic sealing unit configured for carrying out ultrasonic welding of the overlapped side edges 24, 26. Similar results could be obtained with a laser or mechanical sealing unit.

The apparatus 32 may comprise a linear folding unit 76 configured for folding side portions of the finished absorbent garments 10 as shown in figure 10.

The apparatus 32 may comprise a final press unit 78 configured for pressing the folded portions of the finished absorbent garments 10.

The apparatus 32 may comprise a final folding unit 80 configured for carrying out a final folding of the finished absorbent garments 10 as shown in figure 11. After the final folding unit 80 the finished absorbent garments 10 may be sent to a packaging machine.

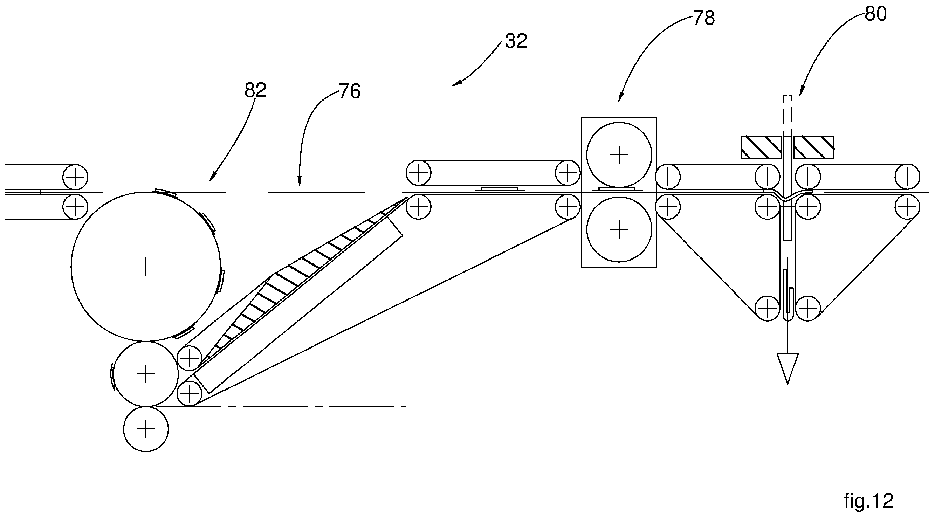

Figure 12 shows an embodiment of the apparatus 32 alternative to the embodiment of figure 5. The elements corresponding to those previously described are indicated by the same reference numbers.

In the embodiment of figure 12 the side edges 24, 26 of the rear and front waist sections 14, 16 of the blank absorbent garments 70 are fixed to each other by glue. The glue dispenser 56 (figure 4) may be configured for applying discrete glue layers also on the portions of the first continuous fabric layer 36 which will form the side edges 24, 26 of the rear and front waist sections 14, 16 of the blank absorbent garments 70. In the embodiment of figure 12 the apparatus 32 comprises a folding and press unit 82 which receives the folded blank absorbent garments 70 from the folding unit 72. The press unit 82 is configured to fold and press the side edges 24, 26 of the rear and front waist sections 14, 16 for glue sealing the side edges 24, 26.

The remaining units 76, 78, 80 of the embodiment of figure 12 are the same as the corresponding units of the embodiment of figure 5.

In operation, the apparatus 32 implements a method for producing washable absorbent garments 10, comprising:
- providing a first continuous fabric layer 36 movable in a longitudinal direction MD,
- applying discrete glue layers on said first continuous fabric layer 36,
- providing a continuous washable absorbent tape 44 containing washable superabsorbent fibers, continuously moving in a longitudinal direction,
- applying to said continuous washable absorbent tape 44 a functionalizing material 31 selected from a group consisting of: graphene, graphene oxide, reduced graphene oxide, and combinations thereof,
- transversely cutting said continuous washable absorbent tape 44 to form individual washable absorbent cores 28,
- spacing from each other in a longitudinal direction said individual washable absorbent cores 28,
- applying said washable absorbent cores 28 on said first continuous fabric layer 36 in longitudinally spaced positions over respective glue layers,
- providing a second continuous fabric layer 50 continuously moving in a longitudinal direction,
- transversely cutting said second continuous fabric layer 50 to form individual top layers 30,
- spacing from each other in a longitudinal direction said individual top layers 30,
- applying said individual top layers 30 over respective washable absorbent cores 28 on said first continuous fabric layer 36, and
- joining said washable absorbent cores 28 and said individual top layers 30 to said first continuous fabric layer 36 by glue to form a continuous washable garment tape (59), and
- transversely cutting said continuous washable garment tape 59 to form individual blank absorbent garments 70.

The method and apparatus according to the present invention may have a production rate up to 200 pieces/1' , which is a huge progress as compared to the production rate of prior art washable absorbent products which is about 1 piece/1' .

Of course, without prejudice to the principle of the invention, the details of construction and the embodiments can be widely varied with respect to those described and illustrated, without thereby departing from the scope of the invention as defined by the claims that follow.

## Claims

1. A washable absorbent garment (10) comprising:
- a back layer (12) of washable fabric forming a pant structure and having rear and front waist sections (14, 16) and a crotch section (18) intermediate between the rear and front waist sections (14, 16), wherein the rear and front waist sections (14, 16) have respective side edges (24, 26) joined to each other,
- a washable absorbent core (28) including a washable absorbent pad comprising washable superabsorbent fibers, and
- a top layer (30) of washable fabric which covers the washable absorbent core (28),
**characterized in that** the washable absorbent core (28) comprises a functionalizing material (31) selected from a group consisting of: graphene, graphene oxide, reduced graphene oxide, and combinations thereof.

2. The washable absorbent garment of claim 1, wherein said functionalizing material (31) is uniformly distributed through the thickness of said washable absorbent core (28).

3. The washable absorbent garment of claim 2, wherein the weight of said functionalizing material (31) distributed through the thickness of said washable absorbent core (28) is comprised in the range 10-250 g/m².

4. The washable absorbent garment of claim 1, wherein said functionalizing material (31) is localized on at least one surface area of said washable absorbent core (28) adjacent to said top layer (30).

5. The washable absorbent garment of claim 4, wherein the weight of said functionalizing material (31) localized on said surface area is comprised in the range 2-100 g/m².

6. The washable absorbent garment of any of the preceding claims, wherein said functionalizing material (31) is embedded in polymers.

7. The washable absorbent garment of any of the preceding claims, wherein the washable absorbent pad has a thickness comprised between 1 and 7 mm.

8. The washable absorbent garment of any of the preceding claims, wherein the washable absorbent pad is sandwiched between an acquisition and diffusion layer and a barrier film.

9. The washable absorbent garment of any of the preceding claims, wherein the washable absorbent core (28) has a total weight comprised between 150 and 750 g/m².

10. The washable absorbent garment of any of the preceding claims, wherein the washable absorbent core (28) has a total thickness comprised between 1,5 and 8,5 mm.

11. The washable absorbent garment of any of the preceding claims, wherein the washable absorbent core (28) and the top layer (30) extend only on the crotch section (18) of the back layer (12) and do not extend on the rear and front waist sections (16, 18) of the back layer (12).

12. A method for producing washable absorbent garments (10), comprising:
- providing a first continuous fabric layer (36) movable in a longitudinal direction (MD),
- providing a continuous washable absorbent tape (44) containing washable superabsorbent fibers, continuously moving in a longitudinal direction,
- applying to said continuous washable absorbent tape (44) a functionalizing material (31) selected from a group consisting of: graphene, graphene oxide, reduced graphene oxide, and combinations thereof,
- transversely cutting said continuous washable absorbent tape (44) to form individual washable absorbent cores (28),
- spacing from each other in a longitudinal direction said individual washable absorbent cores (28),
- applying said washable absorbent cores (28) on said first continuous fabric layer (36) in longitudinally spaced positions over respective glue layers,
- providing a second continuous fabric layer (50) continuously moving in a longitudinal direction,
- transversely cutting said second continuous fabric layer (50) to form individual top layers (30),
- spacing from each other in a longitudinal direction said individual top layers (30),
- applying said individual top layers (30) over respective washable absorbent cores (28) on said first continuous fabric layer (36),
- permanently attaching said washable absorbent cores (28) and said individual top layers (30) to said first continuous fabric layer (36), and
- transversely cutting said continuous washable garment tape (59) to form individual blank absorbent garments (70).

13. The method of claim 12, wherein said functionalizing material (31) is applied to said continuous washable absorbent tape (44) in the form of powder or in the form of a liquid suspension.

14. An apparatus for producing washable absorbent garments (10), comprising:
- a first unwinding unit (34) configured for unwinding a first continuous fabric layer (36) from a first reel (38),
- a second unwinding unit (42) configured for unwinding a continuous washable absorbent tape (44) from a second reel (46),
- an application device (33) configured for applying a functionalizing material (31) selected from a group consisting of: graphene, graphene oxide, reduced graphene oxide, and combinations thereof to said continuous washable absorbent tape (44),
- a first cut-and-slip unit (48) configured for transversally cutting the continuous washable absorbent tape (44) to form individual washable absorbent cores (28), wherein the first cut-and-slip unit (48) is configured for longitudinally spacing from each other the individual absorbent cores (28) and for applying the individual absorbent cores (28) on the first continuous fabric layer (36) in longitudinally spaced positions,
- a third unwinding unit (49) configured for unwinding a second continuous fabric layer (50) from a third reel (52),
- a second cut-and-slip unit (54) configured for transversally cutting the second continuous fabric layer (50) to form individual top layers (30), wherein the second cut-and-slip unit (54) is configured for longitudinally spacing from each other the individual top layers (30) and for applying the individual top layers (30) on respective absorbent cores (28) previously applied on the first continuous fabric layer (36),
- an ultrasonic welding unit or a glue dispenser (56) configured for permanently attaching by ultrasonic welding or by glue the individual absorbent cores (28) and the individual top layers (30) on the first continuous fabric layer (36).

15. The apparatus of claim 14, wherein said application device (33) is configured for applying said functionalizing material (31) in the form of powder or in the form of a liquid suspension.

## Patentansprüche

1. Waschbares absorbierendes Kleidungsstück (10), umfassend:
- eine hintere Schicht (12) aus waschbarem Gewebe, die eine Hosenstruktur bildet und einen hinteren und einen vorderen Taillenabschnitt (14, 16) sowie einen Schrittabschnitt (18) zwischen dem hinteren und dem vorderen Taillenabschnitt (14, 16) aufweist, wobei der hintere und der vordere Taillenabschnitt (14, 16) jeweilige miteinander verbundene Seitenkanten (24, 26) aufweisen,
- einen waschbaren absorbierenden Kern (28) einschließlich eines waschbaren absorbierenden Polsters, das waschbare superabsorbierende Fasern umfasst, und
- eine obere Schicht (30) aus waschbarem Gewebe, die den waschbaren absorbierenden Kern (28) bedeckt,
**dadurch gekennzeichnet, dass** der waschbare absorbierende Kern (28) ein funktionalisierendes Material (31) umfasst, ausgewählt aus einer Gruppe bestehend aus: Graphen, Graphenoxid, reduziertem Graphenoxid und Kombinationen davon.

2. Waschbares absorbierendes Kleidungsstück nach Anspruch 1, wobei das funktionalisierende Material (31) gleichmäßig über die Dicke des waschbaren absorbierenden Kerns (28) verteilt ist.

3. Waschbares absorbierendes Kleidungsstück nach Anspruch 2, wobei das Gewicht des funktionalisierenden Materials (31), das über die Dicke des waschbaren absorbierenden Kerns (28) verteilt ist, im Bereich von 10-250 g/m² umfasst ist.

4. Waschbares absorbierendes Kleidungsstück nach Anspruch 1, wobei das funktionalisierende Material (31) auf mindestens einem Oberflächenbereich des waschbaren absorbierenden Kerns (28) neben der oberen Schicht (30) lokalisiert ist.

5. Waschbares absorbierendes Kleidungsstück nach Anspruch 4, wobei das Gewicht des auf dem Oberflächenbereich lokalisierten funktionalisierenden Materials (31) im Bereich von 2-100 g/m² umfasst ist.

6. Waschbares absorbierendes Kleidungsstück nach einem der vorstehenden Ansprüche, wobei das funktionalisierende Material (31) in Polymere eingebettet ist.

7. Waschbares absorbierendes Kleidungsstück nach einem der vorstehenden Ansprüche, wobei das waschbare absorbierende Polster eine Dicke aufweist, die zwischen 1 und 7 mm umfasst ist.

8. Waschbares absorbierendes Kleidungsstück nach einem der vorstehenden Ansprüche, wobei das waschbare absorbierende Polster zwischen einer Aufnahme- und Diffusionsschicht und einer Barrierefolie eingeschoben ist.

9. Waschbares absorbierendes Kleidungsstück nach einem der vorstehenden Ansprüche, wobei der waschbare absorbierende Kern (28) ein Gesamtgewicht aufweist, das zwischen 150 und 750 g/m² umfasst ist.

10. Waschbares absorbierendes Kleidungsstück nach einem der vorstehenden Ansprüche, wobei der waschbare absorbierende Kern (28) eine Gesamtdicke aufweist, die zwischen 1,5 und 8,5 mm umfasst ist.

11. Waschbares absorbierendes Kleidungsstück nach einem der vorstehenden Ansprüche, wobei sich der waschbare absorbierende Kern (28) und die obere Schicht (30) nur auf dem Schrittabschnitt (18) der hinteren Schicht (12) erstrecken und sich nicht auf dem hinteren und dem vorderen Taillenabschnitt (16, 18) der hinteren Schicht (12) erstrecken.

12. Verfahren zur Herstellung waschbarer absorbierender Kleidungsstücke (10), umfassend:
- Bereitstellen einer ersten durchgehenden Gewebeschicht (36), die in einer Längsrichtung (MD) bewegbar ist,
- Bereitstellen eines durchgehenden waschbaren absorbierenden Bandes (44), das waschbare superabsorbierende Fasern enthält und sich kontinuierlich in Längsrichtung bewegt,
- Aufbringen, auf das durchgehende waschbare absorbierende Band (44), eines funktionalisierenden Materials (31), ausgewählt aus der Gruppe bestehend aus: Graphen, Graphenoxid, reduziertem Graphenoxid und Kombinationen davon,
- Querschneiden des durchgehenden waschbaren absorbierenden Bandes (44), um einzelne waschbare absorbierende Kerne (28) zu bilden,
- Beabstanden der einzelnen waschbaren absorbierenden Kerne (28) voneinander in einer Längsrichtung,
- Aufbringen der waschbaren absorbierenden Kerne (28) auf der ersten durchgehenden Gewebeschicht (36) in in Längsrichtung beabstandeten Positionen über jeweiligen Klebeschichten,
- Bereitstellen einer zweiten durchgehenden Gewebeschicht (50), die sich kontinuierlich in Längsrichtung bewegt,
- Querschneiden der zweiten durchgehenden Gewebeschicht (50), um einzelne obere Schichten (30) zu bilden,
- Beabstanden der einzelnen oberen Schichten (30) voneinander in Längsrichtung,
- Aufbringen der einzelnen oberen Schichten (30) über jeweiligen waschbaren absorbierenden Kernen (28) auf der ersten durchgehenden Gewebeschicht (36),
- dauerhaftes Anbringen der waschbaren absorbierenden Kerne (28) und der einzelnen oberen Schichten (30) an der ersten durchgehenden Gewebeschicht (36), und
- Querschneiden des durchgehenden waschbaren Bekleidungsbandes (59), um einzelne unbedruckte absorbierende Kleidungsstücke (70) zu bilden.

13. Verfahren nach Anspruch 12, wobei das funktionalisierende Material (31) in der Form eines Pulvers oder in der Form einer flüssigen Suspension auf das durchgehende waschbare absorbierende Band (44) aufgebracht wird.

14. Vorrichtung zur Herstellung waschbarer absorbierender Kleidungsstücke (10), umfassend:
- eine erste Abwickeleinheit (34), die zum Abwickeln einer ersten durchgehenden Gewebeschicht (36) von einer ersten Spule (38) konfiguriert ist,
- eine zweite Abwickeleinheit (42), die zum Abwickeln eines durchgehenden waschbaren absorbierenden Bandes (44) von einer zweiten Spule (46) konfiguriert ist,
- eine Aufbringvorrichtung (33), die zum Aufbringen eines funktionalisierenden Materials (31) konfiguriert ist, ausgewählt aus einer Gruppe bestehend aus: Graphen, Graphenoxid, reduziertem Graphenoxid und Kombinationen davon zu dem durchgehenden waschbaren absorbierenden Band (44),
- eine erste Schneid-und-Gleit-Einheit (48), die zum Querschneiden des durchgehenden waschbaren absorbierenden Bandes (44) konfiguriert ist, um einzelne waschbare absorbierende Kerne (28) zu bilden, wobei die erste Schneid-und-Gleit-Einheit (48) dazu konfiguriert ist, die einzelnen absorbierenden Kerne (28) in Längsrichtung voneinander zu beabstanden und die einzelnen absorbierenden Kerne (28) in in Längsrichtung beabstandeten Positionen auf die erste durchgehende Gewebeschicht (36) aufzubringen,
- eine dritte Abwickeleinheit (49), die zum Abwickeln einer zweiten durchgehenden Gewebeschicht (50) von einer dritten Spule (52) konfiguriert ist,
- eine zweite Schneid-und-Gleit-Einheit (54), die zum Querschneiden der zweiten durchgehenden Gewebeschicht (50) konfiguriert ist, um einzelne obere Schichten (30) zu bilden, wobei die zweite Schneid-und-Gleit-Einheit (54) dazu konfiguriert ist, die einzelnen oberen Schichten (30) in Längsrichtung voneinander zu beabstanden und die einzelnen oberen Schichten (30) auf jeweilige absorbierende Kerne (28) aufzubringen, die zuvor auf der ersten durchgehenden Gewebeschicht (36) aufgebracht wurden,
- eine Ultraschallschweißeinheit oder einen Kleberspender (56), der zum dauerhaften Anbringen der einzelnen absorbierenden Kerne (28) und der einzelnen oberen Schichten (30) durch Ultraschallschweißen oder durch Kleber auf der ersten durchgehenden Gewebeschicht (36) konfiguriert ist.

15. Vorrichtung nach Anspruch 14, wobei die Aufbringvorrichtung (33) zum Aufbringen des funktionalisierenden Materials (31) in der Form eines Pulvers oder in der Form einer flüssigen Suspension konfiguriert ist.

## Revendications

1. Vêtement (10) absorbant lavable comprenant :
- une couche arrière (12) de tissu lavable formant une structure de culotte et présentant des sections (14, 16) de taille avant et arrière et une section (18) d'entrejambe intermédiaire entre les sections (14, 16) de taille avant et arrière, dans lequel les sections (14, 16) de taille avant et arrière présentent des bords latéraux (24, 26) respectifs reliés entre eux,
- une partie centrale (28) absorbante lavable incluant un coussinet absorbant lavable comprenant des fibres superabsorbantes lavables, et
- une couche supérieure (30) de tissu lavable qui recouvre la partie centrale (28) absorbante lavable,
**caractérisé en ce que** la partie centrale (28) absorbante lavable comprend un matériau (31) de fonctionnalisation sélectionné parmi un groupe constitué des éléments suivants : graphène, oxyde de graphène, oxyde de graphène réduit et combinaisons de ceux-ci.

2. Vêtement absorbant lavable selon la revendication 1, dans lequel ledit matériau (31) de fonctionnalisation est uniformément réparti à travers l'épaisseur de ladite partie centrale (28) absorbante lavable.

3. Vêtement absorbant lavable selon la revendication 2, dans lequel le poids dudit matériau (31) de fonctionnalisation réparti à travers l'épaisseur de ladite partie centrale (28) absorbante lavable est compris dans la plage de 10 à 250 g/m².

4. Vêtement absorbant lavable selon la revendication 1, dans lequel ledit matériau (31) de fonctionnalisation est situé sur au moins une zone de surface de ladite partie centrale (28) absorbante lavable adjacente à ladite couche supérieure (30).

5. Vêtement absorbant lavable selon la revendication 4, dans lequel le poids dudit matériau (31) de fonctionnalisation situé sur ladite zone de surface est compris dans la plage allant de 2 à 100 g/m².

6. Vêtement absorbant lavable selon l'une quelconque des revendications précédentes, dans lequel ledit matériau (31) de fonctionnalisation est intégré dans des polymères.

7. Vêtement absorbant lavable selon l'une quelconque des revendications précédentes, dans lequel le coussinet absorbant lavable présente une épaisseur comprise entre 1 et 7 mm.

8. Vêtement absorbant lavable selon l'une quelconque des revendications précédentes, dans lequel le coussinet absorbant lavable est pris en sandwich entre une couche d'acquisition et de diffusion et un film barrière.

9. Vêtement absorbant lavable selon l'une quelconque des revendications précédentes, dans lequel la partie centrale (28) absorbante lavable présente un poids total compris entre 150 et 750 g/m².

10. Vêtement absorbant lavable selon l'une quelconque des revendications précédentes, dans lequel la partie centrale (28) absorbante lavable présente une épaisseur totale comprise entre 1,5 et 8,5 mm.

11. Vêtement absorbant lavable selon l'une quelconque des revendications précédentes, dans lequel la partie centrale (28) absorbante lavable et la couche supérieure (30) s'étendent uniquement sur la section (18) d'entrejambe de la couche arrière (12) et ne s'étendent pas sur les sections (16, 18) de taille arrière et avant de la couche arrière (12).

12. Procédé de production de vêtements (10) absorbants lavables, comprenant :
- une fourniture d'une première couche (36) de tissu continue pouvant se déplacer dans une direction longitudinale (MD),
- une fourniture d'une bande (44) absorbante lavable continue contenant des fibres superabsorbantes lavables, se déplaçant en continu dans une direction longitudinale,
- une application, sur ladite bande (44) absorbante lavable continue, d'un matériau (31) de fonctionnalisation sélectionné parmi un groupe constitué des éléments suivants : graphène, oxyde de graphène, oxyde de graphène réduit et combinaisons de ceux-ci,
- une découpe transversale de ladite bande (44) absorbante lavable continue pour former des parties centrales (28) absorbantes lavables individuelles,
- un espacement, dans une direction longitudinale, desdites parties centrales (28) absorbantes lavables individuelles les unes par rapport aux autres,
- une application desdites parties centrales (28) absorbantes lavables sur ladite première couche (36) de tissu continue dans des positions espacées longitudinalement sur des couches de colle respectives,
- une fourniture d'une deuxième couche (50) de tissu continue pouvant se déplacer en continu dans une direction longitudinale,
- une découpe transversale de ladite deuxième couche (50) de tissu continue pour former des couches supérieures individuelles (30),
- un espacement, dans une direction longitudinale, desdites couches supérieures individuelles (30) les unes par rapport aux autres,
- une application desdites couches supérieures individuelles (30) sur des parties centrales (28) absorbantes lavables respectives sur ladite première couche (36) de tissu continue,
- une fixation permanente desdites parties centrales (28) absorbantes lavables et desdites couches supérieures individuelles (30) sur ladite première couche (36) de tissu continue, et
- une découpe transversale de ladite bande (59) de vêtement lavable continue pour former des vêtements (70) absorbants vierges individuels.

13. Procédé selon la revendication 12, dans lequel ledit matériau (31) de fonctionnalisation est appliqué sur ladite bande (44) absorbante lavable continue sous la forme d'une poudre ou sous la forme d'une suspension liquide.

14. Appareil de production de vêtements (10) absorbants lavables, comprenant :
- une première unité (34) de déroulement configurée pour dérouler une première couche (36) de tissu continue à partir d'une première bobine (38),
- une deuxième unité (42) de déroulement configurée pour dérouler une bande (44) absorbante lavable continue à partir d'une deuxième bobine (46),
- un dispositif (33) d'application configuré pour appliquer un matériau (31) de fonctionnalisation sélectionné parmi un groupe constitué des éléments suivants : graphène, oxyde de graphène, oxyde de graphène réduit et combinaisons de ceux-ci, sur ladite bande (44) absorbante lavable continue,
- une première unité (48) de découpe et de glissement configurée pour découper transversalement la bande (44) absorbante lavable continue pour former des parties centrales (28) absorbantes lavables individuelles, dans lequel la première unité (48) de découpe et de glissement est configurée pour espacer longitudinalement les parties centrales (28) absorbantes individuelles les unes par rapport aux autres et pour appliquer les parties centrales (28) absorbantes individuelles sur la première couche (36) de tissu continue dans des positions espacées longitudinalement,
- une troisième unité (49) de déroulement configurée pour dérouler une deuxième couche (50) de tissu continue à partir d'une troisième bobine (52),
- une deuxième unité (54) de découpe et de glissement configurée pour découper transversalement la deuxième couche (50) de tissu continue pour former des couches supérieures individuelles (30), dans lequel la deuxième unité (54) de découpe et de glissement est configurée pour espacer longitudinalement les couches supérieures individuelles (30) les unes par rapport aux autres et pour appliquer les couches supérieures individuelles (30) sur des parties centrales (28) absorbantes respectives précédemment appliquées sur la première couche (36) de tissu continue,
- une unité de soudage par ultrasons ou un distributeur (56) de colle configuré(e) pour fixer de façon permanente par soudage par ultrasons ou à l'aide de colle les parties centrales (28) absorbantes individuelles et les couches supérieures individuelles (30) sur la première couche (36) de tissu continue.

15. Appareil selon la revendication 14, dans lequel ledit dispositif (33) d'application est configuré pour appliquer ledit matériau (31) de fonctionnalisation sous la forme d'une poudre ou sous la forme d'une suspension liquide.
